(19) Europäisches Patentamt
European Patent Office
Office européen des brevets

(11) Publication number: **0 108 654**
**B1**

(12) # EUROPEAN PATENT SPECIFICATION

(45) Date of publication of patent specification: **03.06.87**

(21) Application number: **83401749.3**

(22) Date of filing: **05.09.83**

(51) Int. Cl.$^4$: **C 07 C 143/22,**
C 07 C 143/38, C 08 F 8/36,
C 08 F 32/00, C 08 G 16/02,
C 04 B 24/16

(54) **Sulfonic acid compound having cyclopentadiene skeleton and composition comprising same and cement.**

(30) Priority: **04.10.82 JP 174400/82**
**06.10.82 JP 175666/82**

(43) Date of publication of application:
**16.05.84 Bulletin 84/20**

(45) Publication of the grant of the patent:
**03.06.87 Bulletin 87/23**

(84) Designated Contracting States:
**FR IT**

(56) References cited:
**GB-A-1 437 044**
**GB-A-1 497 317**
**GB-A-1 536 526**

**CHEMICAL ABSTRACTS, vol. 52, January 1958, column 368 c-e, Columbus, Ohio, US, A.N. NESMEYANOV et al.: "Ferrocenesulfonic acids"**

**CHEMICAL ABSTRACTS, vol. 54, January 10, 1960, page 1960, column 469 c-f, Columbus, Ohio, US, A.N. NESMEYANOV et al.: "Derivatives of ferrocene-1-carboxy-1'-sulfonic acid"**

**CHEMICAL ABSTRACTS, vol. 73, August 3, 1970, no. 24625r, Columbus, Ohio, US, V. SETKINA et al.: "Orienting action of the sulfo**

(73) Proprietor: **JAPAN SYNTHETIC RUBBER CO., LTD.**
**11-24, Tsukiji-2-chome Chuo-ku**
**Tokyo 104 (JP)**

(72) Inventor: **Shinohara, Hironobu**
**1, Morigayamacho**
**Yokkaichi-shi (JP)**
Inventor: **Yamahara, Noboru**
**1, Morigayamacho**
**Yokkaichi-shi (JP)**
Inventor: **Yoshida, Yoshinori**
**20-21, Hinaganishi-3-chome**
**Yokkaichi-shi (JP)**

(74) Representative: **Polus, Camille et al**
**c/o Cabinet Lavoix 2, Place d'Estienne d'Orves**
**F-75441 Paris Cedex 09 (FR)**

(56) References cited:
**group on a cyclopentadienylmanganese tricarbonyl system in the protophilic isotopic exchange of hydrogen"**

**TETRAHEDRON LETTERS, vol. 32, 1974, pages 2743-2746, Pergamon Press, London, GB, J.M. SHEPHERD et al.: "An alkali induced 1,4-hydride shift in endo-tricyclo (5.2.1.0(2,6))decyl ketols"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 108 654 B1

Courier Press, Leamington Spa, England.

**Description**

This invention relates to compounds having a cyclopentadiene skeleton and at least one sulfonic acid group.

In general, sulfonic acid and its derivatives, which are organic compounds, are strong acids comparable with sulfuric acid, and are industrially used in a wide field taking advantage of their properties. And their salts are water-soluble and hence very important as surfactants for organic or inorganic materials.

However, many of sulfonation products which have heretofore been synthesized are those of aromatic or aliphatic compounds, and substantially no sulfonation products of alicyclic compounds are known.

The present inventors have conducted extensive research on sulfonation products obtained by using alicyclic compounds and their derivatives as starting materials to find that compounds having a cyclopentadiene skeleton and at least one sulfonic acid group can be produced, and that the obtained compounds or their salts are water-soluble, and hence have an excellent surface-active effect on organic and inorganic materials and have particularly excellent effect on the dispersing of cement in water.

According to this invention, there is provided a sulfonation product of a cyclopentadiene derivative represented by the formula (A) or (B), or a sulfonation product of a polymer of a cyclopentadiene derivative represented by the formula (A) or (B):

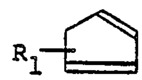

(A)

or

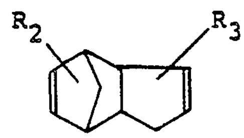

(B)

wherein $R_1$ is a hydrogen atom or an alkyl group having 1 to 3 carbon atoms, and $R_2$ and $R_3$, which may be identical or different, are hydrogen atoms or alkyl groups having 1 to 3 carbon atoms.

The sulfonation products include the following:

(1) Sulfonation products of polymers of cyclopentadiene derivatives represented by the formula (A) or (B)

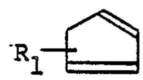

(A)

wherein $R_1$ is a hydrogen atom or an alkyl group having 1 to 3 carbon atoms,

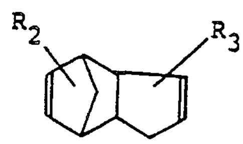

(B)

wherein $R_2$ and $R_3$, which may be the same or different, represent hydrogen atoms or alkyl groups having 1 to 3 carbon atoms.

(2) Sulfonated dicylopentadienes represented by the formula (F):

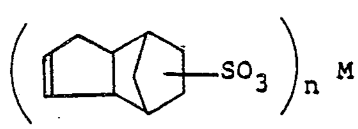

(C)

wherein M is a hydrogen atom, an alkali metal, an alkaline earth metal, ammonium or an amine; and n is 1 or 2 and when M is an alkaline earth metal n is 2.

(3) Polymers or copolymers of sulfonated dicyclopentadienes represented by the formula (C).

Concrete examples of cyclopentadiene derivatives represented by the formula (A) or (B) used in the above sulfonation products (1) include, for instance, cyclopentadiene; alkylcyclopentadienes such as methylcyclopentadiene, ethylcyclopentadiene, propylcyclopentadiene and the like; and dimers consisting

of any combination thereof, such as dicyclopentadiene, and the like. In this invention, these cyclopentadiene derivatives may be used alone or in combination of two or more different derivatives. Among them, cyclopentadiene, dicyclopentadiene and a mixture of the two are preferred.

The cyclopentadiene derivative used in this invention may contain impurities so long as the reaction is not hindered thereby.

Polymerizable monomers other than the cyclopentadiene derivatives may be used in the production of the aforesaid polymers in this invention (hereinafter referred to merely as "the polymerizable monomers"), and as the polymerizable monomers, there may be used hydrocarbon compounds having at least one olefinic double bond in the molecule, including aliphatic, alicylic and aromatic ones. In order to sulfonate the portion of said polymerizable monomer in the polymers, it is necessary that at least one double bond should remain in the said portion. Therefore, the hydrocarbon compounds should preferably have at least two double bonds in the molecule, as in, for example, dienes. However, aliphatic dienes are not preferable for the purpose of increasing the strength of cement because they cause a large decrease of the surface tension of an aqueous solution of the resulting sulfonation product and also cause an increase of the air-entraining effect when used as dispersants for cement. In the case of using the polymerizable monomer, the amount thereof is such that the proportion of the cyclopentadiene derivative to the polymerizable monomer is preferably 20% by weight or more, more preferably 50% by weight or more.

An acidic compound catalyst is used in the production of the aforesaid polymers in this invention, and as the acidic compound in this case, there may be used, for example, Lewis acids such as sulfuric acid, phosphoric acid, hydrogen fluoride, boron trifluoride, boron trifluoride complexes, aluminium chloride, aluminium bromide, tin tetrachloride, zinc chloride, titanium trichloride, and the like or organic protonic acids.

In the presence of such a catalyst, the cyclopentadiene derivative is usually subjected, alone or together with the polymerizable monomer, to polymerization at a reaction temperature of −20°C to 150°C for several hours to obtain a polymer. In this polymerization, a polymerization solvent for allowing the reaction to proceed smoothly may be used, and as such a polymerization solvent, any solvent such as a hydrocarbon, a halogenated hydrocarbon or the like may be used so long as the polymerization is not hindered.

The number average molecular weight of the aforesaid polymer may properly be varied depending upon the reaction conditions, in particular, the kind and amount of the acidic compound catalyst and the reaction temperature, though it is preferably 200 or more when the sulfonation product of this invention is used as the dispersant for cement described below, and it is also preferable that the number average molecular weight is 10,000 or less from the viewpoint of facilitating the sulfonation of said polymers. The number average molecular weight is particularly preferably 300 to 5,000.

The number of double bonds remaining in the aforesaid polymer (hereinafter referred to merely as "the residual double bonds") can be determined by, for example, iodometry, and, in usual, they remain in a proportion of 0.3 to 1 double bond per molecule of the cyclopentadiene derivative.

For sulfonating the polymer thus obtained, there may be used the methods described in detail in E.E. Gilbert, "Sulfonation and Related Reaction", Interscience Publishers Inc. (1965), and a sulfonation method applicable to unsaturated compounds, in particular, unsaturated aliphatic or alicyclic compounds, may properly be selected depending upon the conditions of a reaction system.

The sulfonation products can also be obtained by the addition reaction of a sulfite to unsaturation shown in Charles J. Norton, "the Journal of Organic Chemistry", 4158 (1968). As the sulfonating agents, in this case, there are usually used acidic sulfites, metalsulfites or sulfites of alkali metals alone or in admixture of two or more. The amount of the sulfonating agent may vary depending upon the required degree of sulfonation, and cannot unconditionally be determined, but in usual, they are used in a proportion of 0.1 to 10 molecules per one residual double bond in the polymer.

In the sulfonation, the employment of a catalyst is not always required, but in usual, the reaction time can be shortened when a catalyst such as an inorganic oxidizing agent or the like is used. As the inorganic oxidizing agent, there may be used, for example, nitrates, nitrites, chlorates and the like, and nitrates are particularly preferred.

Is is preferable to use a suitable solvent in order to allow the reaction to proceed uniformly and smoothly. As said solvent, there may advantageously be used, for example, water, lower alchols such as methyl alcohol, ethyl alcohol, propyl alcohol, isopropyl alcohol, butyl alcohol, tertiary butyl alcohol and the like, lower glycols, ketones, ethers, esters, etc. These solvents may properly be used in admixture of two of more. Among them, a mixed solvent of a lower alcohol and water, in particular, a mixed solvent of propyl alcohol and water, is recommended as an excellent solvent.

In order to dissolve the aforesaid polymer more uniformly, there may be co-used another solvent inert to the sulfonation, for example, an aromatic hydrocarbon such as benzene, toluene, xylene, ethylbenzene or the like; an aliphatic hydrocarbon such as pentane, heptane, decane or the like; or a cylic ether such as tetrahydrofuran or the like.

The reaction temperature in the sulfonation is usually 50°C to 200°C, preferably 70°C to 150°C, more preferably 90°C to 120°C, and the reaction can be effected either at atmospheric pressure or under pressure.

In order to suppress the progress of side reactions and inhibit the production of unnecessary inorganic salt, the pH of the reaction system is usually kept at 2 to 9, preferably 5 to 7.

In the sulfonation products thus obtained, 20 to 100% of the residual double bonds in the aforesaid polymers have been sulfonated. The degree of the sulfonation can be obtained by converting the obtained sulfonation product into its acid form by an ion-exchange method, and subjecting the acid form to alkalimetry.

The sulfonation products of this invention can be converted into their acid forms, which can further be converted into their salt forms with an alkali metal, an alkaline earth metal, ammonia, and an amine, by an ion-exchange method, a neutralization method or the like.

For the production of the compounds (2), various production processes are conceivable. For example, they can be produced by a process which comprises sulfonating dicyclopentadiene represented by the formula (L) shown below, and then, if necessary, converting it into a sulfonate:

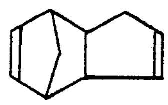

(L)

As to the sulfonation applied to unsaturated compounds, in particular, unsaturated aliphatic compounds or unsaturated alicyclic compounds, the method described in above (I) is applied, and although the amount of the sulfonating agent is not critical, it is 0.1 to 2.0 moles, preferably 0.5 to 1.2 moles, more preferably 0.5 to 1.0 mole, per mole of the dicyclopentadiene. When it is less than 0.1 mole, the yield in the reaction becomes low, and when it exceeds 2.0 moles, compounds in which the double bond in the cyclopentadiene ring has also been sulfonated are produced in a large amount.

In the formula (C) for the compound (2), M is hydrogen, alkali metal, amonium, an amine, or an alkaline earth metal.

As the aforesaid alkali metal atom, there may be used sodium, potassium and the like. As the amine, there may be used alkylamines such as methylamine, triethylamine, ethylamine, propylamine, dimethylamine, diethylamine, trimethylamine, triethylamine, butylamine, dibutylamine, tributylamine and the like; polyamines such as ethylenediamine, diethylenetriamine and the like; morpholine; piperidine; etc. As the alkaline earth metals, there may be used calcium, magnesium and the like. These M's are interconvertible into one another by various ion-exchange techniques.

In the case where n is 1, namely M is, for example, Na which is one of the alkali metal, the general formula (C) is represented as follows:

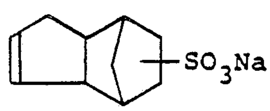

In the case where n is 2, namely M is, for example, Ca which is one of the alkali earth metals, the general formula (C) is represented as follows:

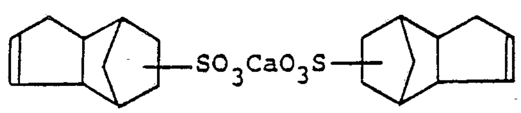

The compound (2) is usually a white to slightly yellow solid, and can be separated from the unreacted organic compounds, for example, by extraction with water. Owing to this procedure, unreacted inorganic salts present in the aqueous phase can be separated by a method such as crystallization or the like.

The sulfonation product thus obtained is a mixture of that having one sulfonic acid group per molecule and that having two sulfonic acid groups per molecule, and the proportions of these components can properly be varied depending upon the kind and amount of the sulfonating agent, the ratio of the sulfonating agent to the dicyclopentadiene, the kind and amount of the inorganic oxidizing agent or the solvent, the reaction temperature, and the like.

The sulfonated dicyclopentadienes may be any of the two kinds of sulfonation products described above. However, from the viewpoint of the facilitation of the polymerization when they are polymerized to obtain a polymer, the sulfonated dicyclopentadiene preferably has residual double bonds and it contains preferably 20% or more of that having one sulfonic acid group per molecule.

The sulfonated dicyclopentadiene contains more preferably 50% or more and most preferably 80% or more of that having one sulfonic acid group per molecule.

The number of sulfonic acid groups of the aforesaid sulfonated dicyclopentadiene can be determined by a usual method such as titration with an alkali. The salts of sulfonic acids include salts with an alkali metal such as sodium, potassium or the like; salts with an alkylamine such as methylamine, ethylamine or the like; salt with an alkaline earth metal such as calcium, magnesium or the like; and ammonium salts.

The compounds (3) are polymers or copolymers of the compounds of the formula (C), and an example of a process for producing them is shown below.

An acidic compound catalyst is used as a polymerization catalyst, and it includes, for example, Lewis acids such as sulfuric acid, phosphoric acid, hydrogen fluoride, boron trifluoride, boron trifluoride complexes, aluminum chloride, aluminum bromide, tin tetrachloride, zinc chloride, titanium trichloride, and the like or organic protonic acids.

In the presence of such a catalyst, the sulfonated dicyclopentadiene (2) alone in admixture with a copolymerizable monomer (hereinafter referred as "the comonomer") is polymerized, in usual, at a reaction temperature of −20° to 300°C, preferably 80° to 180°C for a period of several hours to several tens of hours to obtain a polymer. In the polymerization, a polymerization solvent may be used for effecting the reaction smoothly, and as such a polymerization solvent, there may be used a polar solvent such as water, a hydrocarbon, a halogenated hydrocarbon or the like, so long as the polymerization is not hindered.

It is also possible to control the HLB (surface active effect) by copolymerizing the sulfonation product (2) with the comonomer.

As the comonomer, there may be used, in any proportion, one or more member selected from the group consisting of alphatic, alicyclic and aromatic hydrocarbons having an olefinic unsaturation, olefinically unsaturated amides, olefinically unsaturated alcohols, olefinically unsaturated esters, olefinically unsaturated nitriles, olefinically unsaturated carboxylic acids and esters, olefinically unsaturated sulfonic acids and esters, etc.

However, when the (co-)polymer is used as a dispersant for cement, the content of the sulfonated dicyclopentadiene in the (co-)polymer of this invention is 50% or more, preferably 70% or more, most preferably 90% or more in order to keep the air-entraining property at a low level.

The molecular weight of the (co-)polymer of the aforsesaid sulfonated dicyclopentadiene can properly be controlled by selecting reaction conditions, in particular, the kind and amount of the acidic compound catalyst and the kind and amount of the solvent, the reaction temperature, or the reaction time.

When the polymers or copolymers of the aforesaid sulfonated dicyclopentadienes are used as dispersants for cement, the number average molecular weight of the polymer or copolymer is preferably 500 or more, more preferably 2,000 or more, most preferably 3,500 to 50,000.

The (co-)polymer of the aforesaid sulfonated dicyclopentadiene can be converted into the acid form, which can further be converted to the salt form with an alkali metal, an alkaline earth metal, ammonia, or an amine by an ion-exchange method, neutralization or the like.

Since the compounds (1), (2) and (3) of this invention have an excellent surface active effect, they are very useful as surfactants for organic materials or inorganic materials, and are used as, for example, emulsifiers, dispersants, wetting agents, detergents and smoothing agents. They are particularly useful as dispersants for cement, and in this case, the dispersibility of cement in water can greatly be improved, so that the amount of water can be decreased in a cement construction method.

This invention is further explained below referring to Examples and the accompanying drawings, in which, Fig. 1 shows an infrared absorption spectrum of the sulfonation product obtained in Example 6 and Fig. 2 shows an infrared absorption spectrum of the polymer obtained in Example 7. This invention should not be understood to be limited to the Examples.

The number average molecular weights described in Examples 1, 2, 3 and 4, are obtained by converting the results of measurement by GPC (gel permeation chromatography), by use of a calibration curve obtained by using several polystyrenes different in molecular weight as standard substances.

Example 1

In a 1-liter, three-necked flask equipped with a reflux condenser and a stirrer were placed 400 g of n-hexane and 4 g of a boron trifluoride-phenol complex, and heated to a temperature of 50°C, after which 140 g of dicyclopentadiene having a purity of 95% was added dropwise with stirring over a period of about 1 hour, and the resulting mixture was further subjected to reaction at said temperature for 2 hours. After completion of the reaction, the catalyst was decomposed with an aqueous sodium carbonate solution, and the reaction mixture was washed with water, after which the oil layer was distilled under reduced pressure to remove n-hexane and unreacted dicyclopentadiene. The weight of the resulting residue was 78 g, and the number average molecular weight of the residue was 2,100. The amount of the residual double bonds in the residue was determined by iodometry to find that it was 0.83 equivalent per mole of the reacted dicyclopentadiene.

Subsequently, 20 g of the aforesaid residue, 30 g of toluene, 20 g of sodium hydrogensulfite, 2 g of potassium nitrate, 300 ml of isopropyl alcohol and 50 g of distilled water were placed in a 1-liter, stainless steel autoclave equipped with a stirrer and a thermometer, and air was supplied until the internal pressure in the autoclave became 1.0 $Kg/cm^2$ (gauge pressure) at room temperature. Then, the valve was locked up, and the mixture thus obtained was subjected to reaction at a temperature of 110°C for 5 hours while vigorously stirring the mixture. Thereafter, the reaction mixture was allowed to stand at room temperature, and the major part of the isopropyl alcohol was removed by distillation, after which 1 liter of distilled water and 1.5 liters of petroleum ether were added, and the resulting mixture was sufficiently stirred. The separated petroleum ether layer and the precipitates were removed, and the aqueous layer thus obtained was concentrated to dryness. The concentrate was then dissolved in glacial acetic acid, and an acetic acid-insoluble fraction consisting of an inorganic salt was removed by filtration. The acetic acid-soluble fraction thus obtained was concentrated, to obtain 18.7 g of a whitish-yellow solid. This solid is named "Sample 1".

An aqueous solution of Sample 1 was converted into the acid form by means of an ion-exchange resin and titrated with sodium hydroxide to find that about 78% of the residual double bonds in the residue had been sulfonated. The solubility in water of Sample 1 was 30% by weight or more. Water was added to Sample 1 so as to prepare a 4% by weight aqueous solution, and its surface tension at a temperature of 25°C was measured to find that it was 54.2 dyn/cm.

Example 2

The same treatment as in Example 1 was repeated, except that the reaction was effected at 65°C, to obtain 96 g of a residue. The number average molecular weight of the residue was 530. The amount of the residual double bonds in the residue was determined in the same manner as in Example 1 to find that it was 0.76 equivalent per mole of the reacted dicyclopentadiene.

Subsequently, sulfonation treatment was carried out in the same manner as in Example 1 to obtain 27.3 g of a whitish-yellow solid. This solid is named "Sample 2".

An aqueous solution of Sample 2 was converted into the acid form by means of an ion-exchange resin and titrated with sodium hydroxide to find that about 96% of the residual double bonds in the residue had been sulfonated. The solubility in water of Sample 2 was 40% by weight or more. Water was added to Sample 2 so as to prepare a 4% by weight aqueous solution and its surface tension at a temperature of 25°C was measured to find that it was 51.3 dyn/cm.

Example 3

The same treatment as in Example 1 was repeated, except that the cyclopentadiene was substitued for the dicyclopentadiene and the reaction was effected at a temperature of 30°C, to obtain 68 g of a residue. The number average molecular weight of the residue was 5,600. The amount of the residual double bonds in the residue was determined in the same manner as in Example 1 to find that it was 0.90 equivalent per mole of the reacted cyclopentadiene.

Subsequently, sulfonation treatment was carried out in the same manner as in Example 1 to obtain 14.3 g of a whitish-yellow solid. This solid is named "Sample 3".

An aqueous solution of Sample 3 was converted into the acid form by means of an ion-exchange resin and titrated with sodium hydroxide to find that about 67% of the double bonds in the residue had been sulfonated. The solubility in water of Sample 3 was 20% by weight or more. Water was added to Sample 3 so as to prepare a 4% by weight aqueous solution, and its surface tension at a temperature of 25°C was measured to find that it was 52.3 dyn/cm.

Example 4

The same reaction as in Example 1 was repeated, except that the amount of the dicyclopentadiene was changed from 140 g to 100 g and that 40 g of 1,3-pentadiene having a purity of 70% was newly added and reacted, to obtain 63 g of a residue. The number average molecular weight of the residue was 2,300. The amount of residual double bonds in the residue were determined in the same manner as in Example 1 to find that it was 0.87 equivalent per mole of the total of the reacted dicyclopentadiene and 1,3-pentadiene.

Subsequently, sulfonation treatment was carried out in the same manner as in Example 1, to obtain 17.6 g of a whitish-yellow solid. This solid is named "Sample 4".

An aqueous solution of Sample 4 was converted into the acid form by means of an ion-exchange resin and titrated with sodium hydroxide to find that about 81% of the residual double bonds in the residue had been sulfonated. The solubility in water of Sample 4 was 30% by weight or more. Water was added to Sample 4 so as to prepare a 4% by weight aqueous solution, and the surface tension thereof at a temperature of 25°C was measured to find that it was 49.3 dyn/cm.

Example 5

In 50 g of distilled water was dissolved 2 g of each of Sample 1 to 4 obtained in Examples 1 to 4, whereby four, in total, aqueous solutions were prepared. To each of the aqueous solutions was added 200 g of commercially available portland cement (manufactured by Chichibu Cement Co., Ltd.), and the mixture thus obtained were individually kneaded by hand for 3 minutes, after which the flow values were determined by use of a flow corn with an inner volume of 98.9 cc according to JIS R5201, to obtain the results shown in Table 1.

TABLE 1

| | Flow value (mm) |
|---|---|
| Sample 1 | 157 |
| Sample 2 | 138 |
| Sample 3 | 147 |
| Sample 4 | 150 |

On the other hand, when the same treatment as described above was repeated, except that none of Samples 1 to 4 were added, the flow value was only 87 mm.

As can be understood from Examples 1 to 4, the compounds (1) readily cause foaming and have an excellent surface active effect, and as can be seen from Example 5, when they are used as dispersants for cement, they have very great and excellent effect on dispersing cement in water.

Example 6

A 30-liter, stainless steel autoclave equipped with a closed electromagnetic induction stirrer was used as a reactor. In the autoclave were placed 2,000 g of dicyclopentadiene having a purity of 95%, 1,200 g of sodium hydrogensulfite, 122 g of potassium nitrate, 8,000 ml of isopropyl alcohol and 2,000 ml of distilled water.

Subsequently, the autoclave was completely purged with nitrogen, and then sealed, after which the contents were subjected to reaction at 110°C for 5 hours while strongly stirring the contents. After the reaction mixture was allowed to stand at room temperature, organic salts deposited from the reaction mixture were removed by suction filtration, and the filtrate was concentrated under reduced pressure to a volume of about 4 liters. To the concentrate were added 2.0 liters of distilled water and 1.5 liters of petroleum ether, and the resulting mixture was sufficiently stirred and then subjected to separation whereby unreacted dicyclopentadiene was extracted by the petroleum ether layer and removed.

The residue, i.e., the aqueous layer was concentrated to dryness under reduced pressure to obtain 2,300 g of a whitish-yellow solid. With the solid was mixed 4.0 liters of glacial acetic acid, and an acetic acid-insoluble fraction consisting of inorganic salts such as $NaHSO_3$, $Na_2SO_3$ and the like was separated by means of a centrifuge and removed.

An acetic acid-soluble fraction was concentrated to dryness under reduced pressure to obtain 1,480 g of a light-yellow solid. The light-yellow solid was subjected to extraction with ethanol having a purity of 99.5% by means of a Soxhlet's extractor for 1 hour to extract and remove the residual acetic acid, and then dried. The elementary analysis of the dried solid showed that C = 50.4%; H = 5.3%; S = 14.0% (calculated value: C = 50.8%; H = 5.5%; S = 13.6%). The solid was dissolved in distilled water and freed from Na ion by using a cation-exchange resin, and the amount of sulfonic acid in the thus treated product was measured by titration to find that it was 102% of the calculated value.

As a result of the measurement of infrared absorption spectrum of the treated product by a KBr disc method, absorptions due to cyclopentene ring double bond appeared at 750 $cm^{-1}$ and 1,390 $cm^{-1}$, while an absorption due to norbornene ring double bond at 1570 $cm^{-1}$ observed in the case of dicyclopentadiene disappeared and in place thereof, asorptions due to sulfonic acid group were observed at 1,190 $cm^{-1}$ and 1,050 $cm^{-1}$; therefore it was confirmed that the nonbornene ring double bond had been sulfonated (see Fig. 1).

As a result of the analysis described above, the product was identified as a compound having the structural formula (O):

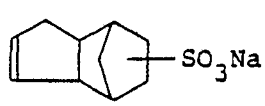

(O)

The surface tension at a temperature of 25°C of a 4% by weight aqueous solution of the compound (O) was measured to find that it was 40 dyn/cm, indicating that the compound had a high surface activity.

The compound (O) was dissolved in distilled water, and freed from Na ion by means of a cation-exchange resin, after which the solution was concentrated to dryness to obtain a solid of the sulfonic acid type of the compound (O). Subsequently, 15 g of the sulfonic acid type of the compound (O) and 6.9 g of sulfuric acid having a purity of 97% were charged into a 300-ml, three-necked flask equipped with a reflux condenser and a stirrer, and subjected to polymerization at a reaction temperature of 120°C for 25 hours with stirring.

After completion of the reaction, the sulfuric acid was removed by procedures of liming and sodation to obtain 15.5 g of the Na salt of a polymer.

The Na salt of a polymer was analyzed by gel permeation chromatography to obtain a graph having the main peak at a position corresponding to a number average molecular weight of 10,000.

The Na salt of a polymer was dissolved in distilled water and freed from Na ion by using a cation-exchange resin, and the amount of sulfonic acid in the thus treated product was measured by titration to find that the treated product had 0.96 sulfonic acid group per one molecule of the dicyclopentadiene.

The surface tension at 25°C of a 4% by weight aqueous solution of the Na salt of a polymer was measured to find that it was 63 dyn/cm.

Subsequently, 2 g of the Na salt of a polymer was dissolved in 50 g of distilled water, and 200 g of Portland cement manufactured by Chichibu Cement Co., Ltd. was added. The resulting mixture was kneaded by hand for 3 minutes, and then subjected to a flow test by use of a flow corn having an inside diameter of 60 mmφ, a height of 35 mm and a capacity of 98.8 $cm^3$ according to the physical test method for cement of JIS R5201, whereby the flow value was determined as 190 mm. The specific gravity of green cement paste in this case was 2.23 $g/cm^3$. On the other hand, after 50 g of distilled water free from the Na salt of a polymer was kneaded with 200 g of Portland cement, the flow value was 87 mm and the specific gravity of green cement paste was 2.24 $g/cm^3$. From this, it can be seen that when the polymer of sulfonated dicyclopentadiene of this invention is used as a dispersant for cement, it has a very great and excellent effect on dispersing cement in water.

Example 7

In 30-liter, stainless steel autoclave equipped with a stirrer and a thermometer were placed 3,000 g of diyclopentadiene, 1,888 g of sodium hydrogensulfite, 91.7 g of potassium nitrate, 12 liters of iospropyl alcohol and 3,000 g of distilled water. Nitrogren was supplied thereto until the internal pressure in the autoclave became 1.0 $kg/cm^2$ (gauge pressure) at room temperature, after which the valve was locked up, and the mixture thus obtained were subjected to reaction at 110°C for 5 hours while strongly stirring the mixture. Thereafter, the reaction mixture was allowed to stand at room temperature, and the major part of the isopropyl alcohol was removed by distillation, after which distilled water and petroleum ether were added, and the resulting mixture was sufficiently stirred. The separated petroleum ether layer and the precipitates were removed, and the aqueous layer thus obtained was concentrated and then evaporated to dryness. The concentrate was then dissolved in glacial acetic acid, and an acetic acid-insoluble fraction consisting of inorganic salts was separated by means of a centrifuge. The acetic acid-soluble fraction thus obtained was concentrated, to obtain 2,800 g of a white solid. The solid is named "Sulfonation Product A"

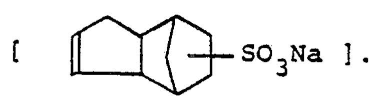

An aqueous solution of Sulfonation Product A was converted into the acid form, afer which the aqueous solution was concentrated to dryness to obtain the acid form of the sulfonation product. This is named "Sulfonation Product B"

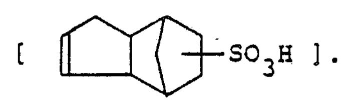

Subsequently, 15 g of Sulfonation Product B and 6.88 g of sulfuric acid were placed in a 300-ml, three-necked flask equipped with a reflux condenser and a stirrer, and subjected to polymerization at 120°C for 26 hours. After completion of the reaction, the reaction mixture was subjected to liming and sodation. The amount of the thus obtained solid was 15.5 g, and the number average molecular weight of this polymer was 10,000. The polymer was named "Sample 10".

Sample 10 was converted into the acid form by means of an ion-exchange resin and titrated with potassium hydroxide to find that the polymer had 0.96 sulfonic acid group per one molecule of dicylopentadiene.

Water was added to Sample 10 so as to prepare a 4% by weight aqueous solution, and the surface tension thereof at a temperature of 25°C was determined as 63 dyn/cm.

The IR chart of Sample 10 is shown in Fig. 2, from which it can be seen that this polymer had sulfonic acid groups (1,190 $cm^{-1}$ and $1{,}050^{-1}$), and that the absorption due to double bond has become weak.

Example 8

The same treatment as in Example 7 was repeated, except that Sulfonation Product A was used, to obtain a polymer having a number average molecular weight of 1,600. The polymer is named "Sample 11".

An aqueous solution of Sample 11 was converted into the acid form by means of an ion-exchange resin and titrated with potassium hydroxide solution to find that the polymer had 0.79 sulfonic acid group per one molecule of dicylopentadiene.

The surface tension of a 4% by weight aqueous solution of Sample 11 was 69.2 dyn/cm.

Example 9

The same procedure as in Example 7 was repeated, except that polymerization was conducted at a temperature of 170°C for 28 hours by using 30 g of Sulfonation Product A, 125 g of sulfuric acid and 11.4 g of distilled water, to obtain a polymer having a number average molecular weight of 8,000. The polymer is named "Sample 12".

The degree of sulfonation was measured in the same manner as in Example 8 to find that Sample 12 had 0.59 sulfonic acid group per one molecule of dicylopentadiene. The surface tension of a 4% by weight aqueous solution of the Sample 12 was 65 dyn/cm.

Example 10

In 50 g of distilled water was dissolved 2 g of each of Samples 10 to 12 to prepare four, in total, aqueous solutions. To each of the aqueous solutions was added 200 g of commercially available portland cement (manufactured by Chichibu Cement Co., Ltd), and the mixture thus obtained was kneaded by hand for 3 minutes, after which the flow value was measured by use of a flow corn with an inner volume of 98.9 cc according to JIS R5201. The results obtained were as shown in Table 2.

TABLE 2

| | Flow value (mm) |
|---|---|
| Sample 10 | 205 |
| Sample 11 | 140 |
| Sample 12 | 170 |

On the other hand, the same treatment as in Example 10 was repeated, except that none of Samples 10 to 12 were added. The flow value in this case was determined as only 87 mm.

As can be seen from Examples 7 to 9, the polymers of the sulfonation product of this invention have an excellent surface active effect, and as can be seen from Example 10, when they are used as dispersants for cement, they have a very great and excellent effect on dispersing cement in water.

Example 11

In a 300-ml, three-necked flask equipped with a reflux condenser and a stirrer were placed 13 g of Sulfonation Product A, 2 g of dicyclopentadiene and 6.88 g of sulfuric acid, and the resulting mixture was subjected to copolymerization at a temperature of 120°C for 20 hours. After the reaction, the reaction mixture was subjected to liming and sodation. The amount of the solid thus obtained was 15.0 g, and the surface tension of a 4% by weight aqueous solution of the obtained copolymer was 40 dyn/cm, namely, the copolymer had an excellent surface active effect.

Example 12

To 500 g of a normal portland cement (manufactured by Chichibu Cement Co., Ltd.), were added 3.5 g of the Na salt of a polymer obtained in Example 6 (dispersant) and 0.35 g of Polymer Emulsion C, as disclosed below after which 125 g of water was added to the resulting mixture. The mixture thus obtained was stirred at a revolution rate of 120 r.p.m. to prepare a cement paste. The flow values of the cement paste were measured in the same manner as mentioned above to obtain the results shown in Table 3.

TABLE 3

| Stirring time (min) | Flow value (mm) |
|---|---|
| 3 | 165 |
| 30 | 160 |
| 60 | 145 |

Example 13

To 500 g of a normal portland cement (manufactured by Chichibu Cement Co., Ltd.), were added 3.5 g of Sample 1 (dispersant) and 0.35 g (as solids) of Polymer Emulsion C, as disclosed below after which 125 g

of water was added to the resulting mixture. The mixture thus obtained was stirred at a revolution rate of 120 r.p.m. to prepare a cement paste. The flow values of the cement paste were measured in the same manner as mentioned above to obtain the results shown in Table 4.

TABLE 4

| Stirring time (min) | Flow value (mm) |
|---|---|
| 3 | 160 |
| 30 | 156 |
| 60 | 131 |

Synthesis of polymer emulsion C

In a 1-liter autoclave were placed 400 g of water, 3 g of sodium dodecylbenzenesulfonate and 0.2 g of potassium persulfate, and polymerization was effected by continuously adding thereto dropwise 200 g of ethyl acrylate at a temperature of 80°C with stirring over a period of 2 hours, whereby a polymer emulsion was synthesized. The degree of polymerization reached 100% in 3 hours after initiation of the polymerization. The polymer emulsion is named "Polymer Emulsion C".

**Claims**

1. A sulfonation product of a cyclopentadiene derivative represented by the formula (A) or (B), or a sulfonation product of a polymer of a cyclopentadiene derivative represented by the formula (A) or (B):

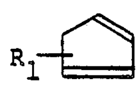

(A)

or

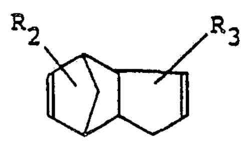

(B)

wherein $R_1$ is a hydrogen atom or an alkyl group having 1 to 3 carbon atoms, and $R_2$ and $R_3$, which may be identical or different, are hydrogen atoms or alkyl groups having 1 to 3 carbon atoms.

2. A compound according to Claim 1, which is a sulfonated dicyclopentadiene represented by the formula:

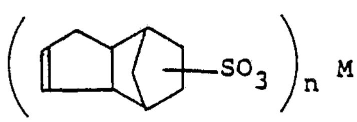

wherein M is a hydrogen atom, an alkali metal, an alkaline earth metal, ammonium or an amine; and n is 1 or 2 and when M is an alkaline earth metal n is 2.

3. A compound according to Claim 1, which is a polymer or copolymer of a sulfonated dicyclopentadiene represented by the formula:

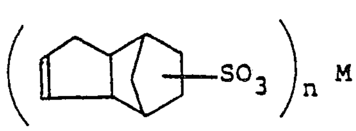

wherein M is a hydrogen atom, an alkali metal, an alkaline earth metal, ammonium or an amine; and n is 1 or 2 and when M is an alkaline earth metal n is 2.

4. A process for producing a polymer or copolymer of a sulfonation product of dicyclopentadiene, which comprises a sulfonated dicyclopentadiene represented by the formula:

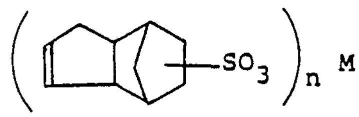

wherein M is a hydrogen atom, an alkali metal, an alkaline earth metal, ammonium or an amine; and n is 1 or 2 and when M is an alkaline earth metal n is 2, by copolymerizing said sulfonated dicyclopentadiene with at least one monomer copolymerizable therewith, in the presence of an acidic compound catalyst.

5. A surfactant for dispersing cement particles in water which comprises a sulfonated dicyclopentadiene or its polymer or copolymer, said sulfonated dicyclopentadiene having the following formula:

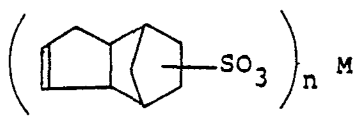

wherein n is 1 or 2 and M is selected from the group consisting of hydrogen, an alkali metal, an alkaline earth metal, ammonia and an amine provided that when M is an alkaline earth metal n is 2.

6. A sulfonation product of a copolymer of a cyclopentadiene derivative represented by the general formula:

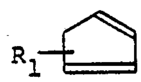

or

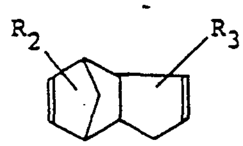

wherein $R_1$ is a hydrogen atom or an alkyl group having 1 to 3 carbon atoms, and $R_2$ and $R_3$, which may be identical or different, are hydrogen atoms or alkyl groups having 1 to 3 carbon atoms, with at least one monomer copolymerizable therewith in such a proportion that the proportion of the sulfonated cyclopentadiene derivative in the copolymer is 70% or more.

7. A process for producing a sulfonation product of a polymer of a cyclopentadiene derivative, which comprises polymerizing a cyclopentadiene derivative represented by the general formula:

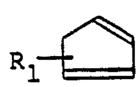

or

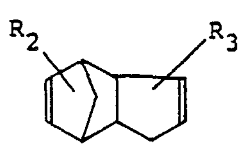

wherein $R_1$ is a hydrogen atom or an alkyl group having 1 to 3 carbon atoms and $R_2$ and $R_3$, which may be identical or different, are hydrogen atoms or alkyl groups having 1 to 3 carbon atoms, in the presence of an acidic compound catalyst, and then sulfonating the resulting polymer.

8. A process for producing a sulfonation product of a copolymer of a cyclopentadiene derivative which comprises copolymerizing a cyclopentadiene derivative of the general formula:

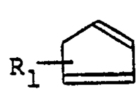

or

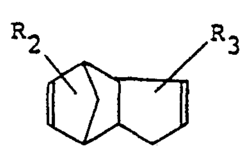

wherein $R_1$ is a hydrogen atom or an alkyl group of 1 to 3 carbon atoms, and $R_2$ and $R_3$, which may be identical or different, are hydrogen atoms or alkyl groups of 1—3 carbon atoms with at least one polymerizable monomer in such a proportion that the proportion of the sulfonated cyclopentadiene derivative in the copolymer is 70% or more, in the presence of an acidic compound catalyst, and then sulfonating the resulting copolymer.

9. A surfactant for dispersing cement particles in water which comprises a sulfonated cyclopentadiene monomer of the formula:

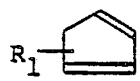

or

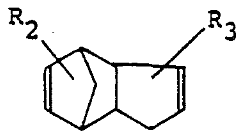

wherein $R_1$ is a hydrogen atom or an alkyl group of 1 to 3 carbon atoms and $R_2$ and $R_3$, which may be the same or different, are hydrogen atoms or alkyl groups of 1 to 3 carbon atoms, or its polymer.

10. A surfactant for dispersing cement particles in water which comprises a sulfonated product of a copolymer of a cyclopentadiene monomer of the formula:

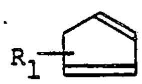

or

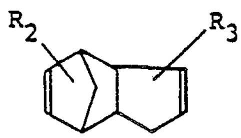

wherein $R_1$ is a hydrogen atom or an alkyl group of 1 to 3 carbon atoms and $R_2$ and $R_3$ which may be the same or different are hydrogen atoms or alkyl groups of 1 to 3 carbon atoms, which copolymer has a proportion of the sulfonated cyclopentadiene of 70% or more.

11. A process for producing a sulfonation product which comprises sulfonating a cyclopentadiene derivative represented by the general formula:

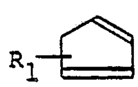

or

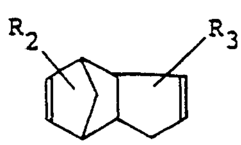

wherein $R_1$ is a hydrogen atom or an alkyl group having 1 to 3 carbon atoms and $R_2$ and $R_3$, which may be the same or different, are hydrogen atoms or alkyl groups having 1 to 3 carbon atoms, in the presence of an acidic compound catalyst.

**Patentansprüche**

1. Sulfonierungsprodukt eines Cyclopentadienderivats der Formel (A) oder (B) oder Sulfonierungsprodukt eines Polymers eines Cyclopentadienderivats der Formel (A) oder (B)

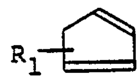
(A)

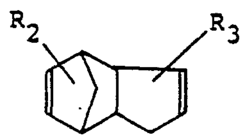
(B)

worin $R_1$ ein Wasserstoffatom oder eine Alkylgruppe mit 1 bis 3 Kohlenstoffatomen und $R_2$ und $R_3$, die gleichartig oder verschieden sein können, Wasserstoffatome oder Alkylgruppen mit 1 bis 3 Kohlenstoffatomen bedeuten.

2. Verbindung nach Anspruch 1, gekennzeichnet durch ein sulfoniertes Dicyclopentadien der Formel

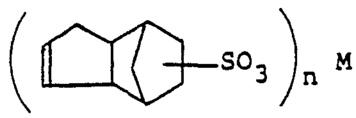

worin M für ein Wasserstoffatom, ein Alkalimetall, ein Erdalkalimetall, Ammonium oder ein Amin und n für 1 oder 2 mit der Maßgabe stehen, daß wenn M ein Erdalkalimetall darstellt, n den Wert 2 besitzt.

3. Verbindung nach Anspruch 1, gekennzeichnet durch ein Polymer oder Copolymer eines sulfonierten Dicyclopentadiens der Formel

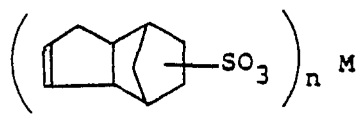

worin M für ein Wasserstoffatom, ein Alkalimetall, ein Erdalkalimetall, Ammonium oder ein Amin und n für 1 oder 2 mit der Maßgabe stehen, daß wenn M ein Erdalkalimetall darstellt, n den Wert 2 besitzt.

4. Verfahren zur Herstellung eines Polymers oder Copolymers eines Sulfonierungsprodukts von Dicyclopentadien, welches ein sulfoniertes Dicyclopentadien der Formel

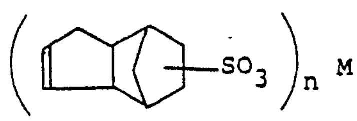

worin M für ein Wasserstoffatom, ein Alkalimetall, ein Erdalkalimetall, Ammonium oder ein Amin und n für 1 oder 2 mit der Maßgabe stehen, daß wenn M ein Erdalkalimetall darstellt, n den Wert 2 besitzt, durch Copolymerisieren des sulfonierten Dicyclopentadiens mit mindestens einem damit copolymerisierbaren Monomer in Gegenwart einer sauren Verbindung als Katalysator.

5. Oberflächenaktives Mittel zum Dispergieren von Zementteilchen in Wasser enthaltend ein sulfoniertes Dicyclopentadien oder ein Polymer oder Copolymer davon, welches sulfonierte Dicyclopentadien der folgenden Formel

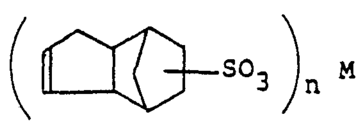

entspricht, worin n 1 oder 2 bedeutet und M aus der Wasserstoff, ein Alkalimetall, ein Erdalkalimetall, Ammonium und ein Amin umfassenden Gruppe ausgewählt ist mit der Maßgabe, daß wenn M ein Erdalkalimetall darstellt, n den Wert 2 besitzt.

6. Sulfonierungsprodukt eines Copolymers aus einem Cyclopentadienderivat der allgemeinen Formel

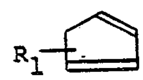

(A)

oder

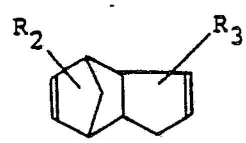

(B)

worin $R_1$ ein Wasserstoffatom oder eine Alkylgruppe mit 1 bis 3 Kohlenstoffatomen und $R_2$ und $R_3$, die gleichartig oder verschieden sein können, Wasserstoffatome oder Alkylgruppen mit 1 bis 3 Kohlenstoffatomen bedeuten, und mindestens einem damit copolymerisierbarem Monomeren in einer solchen Menge, daß der Anteil des sulfonierten Cyclopentadienderivats in dem Copolymer 70% oder mehr beträgt.

7. Verfahren zur Herstellung eines Sulfonierungsprodukts eines Polymers eines Cyclopentadienderivats, dadurch gekennzeichnet, daß man ein Cyclopentadienderivat der allgemeinen Formel

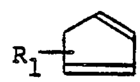

(A)

oder

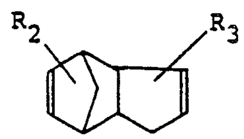

(B)

worin $R_1$ ein Wasserstoffatom oder eine Alkylgruppe mit 1 bis 3 Kohlenstoffatomen und $R_2$ und $R_3$, die gleichartig oder verschieden sein können, Wasserstoffatome oder Alkylgruppen mit 1 bis 3 Kohlenstoffatomen bedeuten, in Gegenwart einer saueren Verbindung als Katalysator polymerisiert und anschließend das gebildete Polymer sulfoniert.

8. Verfahren zur Herstellung eines Sulfonierungsprodukts eines Copolymers eines Cyclopentadienderivats, dadurch gekennzeichnet, daß man ein Cyclopentadienderivat der allgemeinen Formel

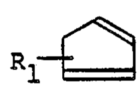

(A)

oder

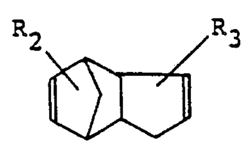

(B)

worin $R_1$ ein Wasserstoffatom oder eine Alkylgruppe mit 1 bis 3 Kohlenstoffatomen und $R_2$ und $R_3$, die gleichartig oder verschieden sein können, Wasserstoffatome oder Alkylgruppen mit 1 bis 3 Kohlenstoffatomen bedeuten, mit mindestens einem polymerisierbaren Monomer in einer solchen Menge, daß der Anteil des sulfonierten Cyclopentadienderivats in dem Copolymer 70% oder mehr beträgt, in Gegenwart einer saueren Verbindung als Katalysator copolymerisiert und dann das gebildete Copolymer sulfoniert.

9. Oberflächenaktives Mittel zum dispergieren von Zementteilchen in Wasser enthaltend ein sulfoniertes Cyclopentadienmonomer der Formel

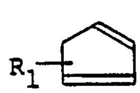

(A)

oder

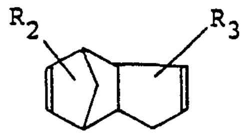

(B)

worin $R_1$ ein Wasserstoffatom oder eine Alkylgruppe mit 1 bis 3 Kohlenstoffatomen und $R_2$ und $R_3$, die gleichartig oder verschieden sein können, Wasserstoffatome oder Alkylgruppen mit 1 bis 3 Kohlenstoffatomen bedeuten, oder ein Polymer davon.

10. Oberflächenaktives Mittel zum Dispergieren von Zementteilchen in Wasser enthaltend ein sulfoniertes Produkt eines Copolymers eines Cyclopentadienmonomers der Formel

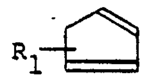

(A)

oder

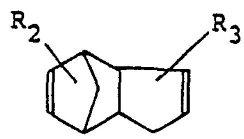

(B)

worin $R_1$ ein Wasserstoffatom oder eine Alkylgruppe mit 1 bis 3 Kohlenstoffatomen und $R_2$ und $R_3$, die gleichartig oder verschieden sein können, Wasserstoffatome oder Alkylgruppen mit 1 bis 3 Kohlenstoffatomen bedeuten, welches Copolymer das sulfonierte Cyclopentadien in einem Anteil von 70% oder mehr enthält.

11. Verfahren zur Herstellung eines Sulfonierungsprodukts, dadurch gekennzeichnet, daß man ein Cyclopentadienderivat der allgemeinen Formel

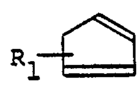

(A)

oder

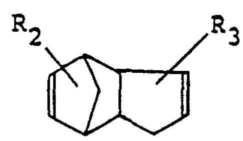

(B)

worin $R_1$ ein Wasserstoffatom oder eine Alkylgruppe mit 1 bis 3 Kohlenstoffatomen und $R_2$ und $R_3$, die gleichartig oder verschieden sein können, Wasserstoffatome oder Alkylgruppen mit 1 bis 3 Kohlenstoffatomen bedeuten, in Gegenwart einer saueren Verbindung als Katalysator sulfoniert.

**Revendications**

1. Produit de sulfonation d'un dérivé de cyclopentadiène représenté par la formule (A) ou (B) ou un produit de sulfonation d'un polymère d'un dérivé de cyclopentadiène représenté par la formule (A) ou (B):

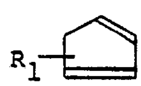

(A)

oder

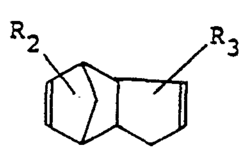

(B)

où $R_1$ est un atome d'hydrogène ou un groupe alkyle ayant 1 à 3 atomes de carbone et $R_2$ et $R_3$, qui peuvent être identiques ou différents, sont des atomes d'hydrogène ou des groupes alkyle ayant 1 à 3 atomes de carbone.

2. Composé selon la revendication 1 qui est un dicyclopentadiène sulfoné représenté par la formule:

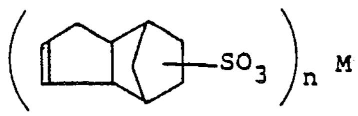

dans laquelle M est un atome d'hydrogène, un métal alcalin, un métal alcalino-terreux, l'ammonium ou une amine; et n est égal à 1 ou 2 et, lorsque M est un métal alcalino-terreux, n est égal à 2.

3. Compose selon la revendication 1 qui est un polymère ou copolymère d'un dicyclopentadiène sulfoné représenté par la formule:

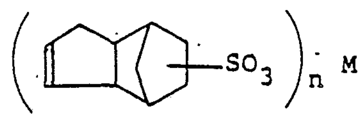

dans laquelle M est un atome d'hydrogène, un métal alcalin, un métal alcalino-terreux, l'ammonium ou une amine; et n est égal à 1 ou 2 et, lorsque M est un métal alcalino-terreux, n est égal à 2.

4. Procédé pour la production d'un polymère ou copolymère d'un produit de sulfonation du dicyclopentadiène qui comprend un dicyclopentadiène sulfoné représenté par la formule:

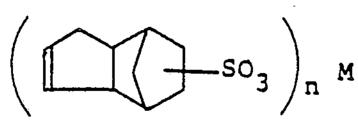

dans laquelle M est un atome d'hydrogène, un métal alcalin, un métal alcalino-terreux, l'ammonium ou une amine; et n est égal à 1 ou 2 et, lorsque M est un métal alcalino-terreux, n est égal à 2, qui consiste à copolymériser ledit dicyclopentadiène sulfoné avec au moins un monomère qui lui est copolymerisable en présence d'un composé catalytique acide.

5. Agent tensio-actif pour la dispersion de particules de ciment dans l'eau qui comprend un dicyclopentadiène sulfoné ou son polymère ou copolymère, ledit dicyclopentadiene sulfoné répondant à la formule suivante:

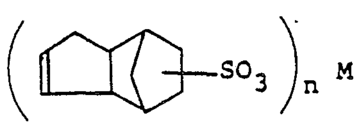

dans laquelle n est égal à 1 ou 2 et M est choisi dans le groupe constitué par l'hydrogène, un métal alcalin, un métal alcalino-terreux, l'ammoniac et une amine, sous réserve que lorsque M est un métal alcalino-terreux, n est égal à 2.

6. Produit de sulfonation d'un copolymère d'un dérivé de cyclopentadiène représenté par la formule générale:

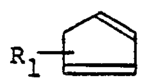

ou

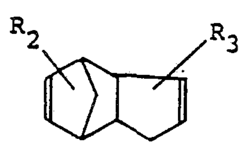

dans laquelle $R_1$ est un atome d'hydrogène ou un groupe alkyle ayant 1 à 3 atomes de carbone et $R_2$ et $R_3$, qui peuvent être identiques ou différents, sont des atomes d'hydrogène ou des groupes alkyle ayant 1 à 3 atomes de carbone, avec au moins un monomère copolymérisable avec lui, en une proportion telle que la proportion du dérivé de cyclopentadiène sulfoné dans le copolymère soit de 70% ou plus.

7. Procédé pour la production d'un produit de sulfonation d'un polymère d'un dérivé de cyclopentadiène qui comprend la polymérisation d'un dérivé de cyclopentadiène représenté par la formule générale:

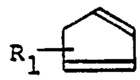

ou

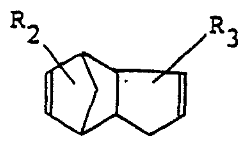

dans laquelle $R_1$ est un atome d'hydrogène ou un groupe alkyle ayant 1 à 3 atomes de carbone et $R_2$ et $R_3$, qui peuvent être identiques ou différents, sont des atomes d'hydrogène ou des groupes alkyle ayant 1 à 3 atomes de carbone, en présence d'un composé catalytique acide, puis la sulfonation du polymère obtenu.

8. Procédé pour la production d'un produit de sulfonation d'un copolymère d'un dérivé de cyclopentadiène qui comprend la copolymérisation d'un dérivé de cyclopentadiène de formule générale:

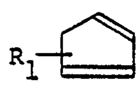

ou

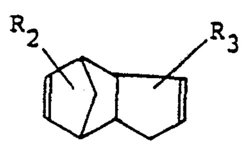

dans laquelle $R_1$ est un atome d'hydrogène ou un groupe alkyle de 1 à 3 atomes de carbone et $R_2$ et $R_3$, qui peuvent être identiques ou différents, sont des atomes d'hydrogène ou des groupes alkyle de 1 à 3 atomes de carbone, avec au moins un monomère polymérisable, en une proportion telle que la proportion du dérivé de cyclopentadiène sulfoné dans le copolymère soit de 70% ou plus, en présence d'un composé catalytique acide, puis la sulfonation du copolymère obtenu.

9. Agent tensio-actif pour disperser des particules de ciment dans l'eau qui comprend un cyclopentadiène sulfoné monomère de formule:

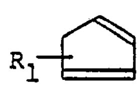

ou

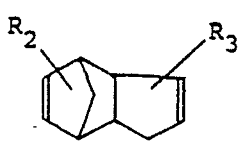

dans laquelle $R_1$ est un atome d'hydrogène ou un groupe alkyle de 1 à 3 atomes de carbone et $R_2$ et $R_3$, qui peuvent être semblables ou différents, sont des atomes d'hydrogène ou des groupes alkyle de 1 à 3 atomes de carbone ou un polymère de celui-ci.

10. Agent tensio-actif pour disperser des particules de ciment dans l'eau qui comprend un produit sulfoné d'un copolymère d'un cyclopentadiène monomère de formule:

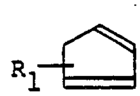

ou

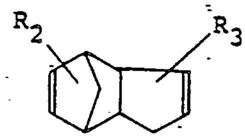

dans laquelle $R_1$ est un atome d'hydrogène ou un groupe alkyle de 1 à 3 atomes de carbone et $R_2$ et $R_3$, qui peuvent être semblables ou différents, sont des atomes d'hydrogène ou des groupes alkyle de 1 à 3 atomes de carbone, lequel copolymère a une proportion du cyclopentadiène sulfoné de 70% ou plus.

11. Procédé pour la production d'un produit de sulfonation qui comprend la sulfonation d'un dérivé de cyclopentadiène représenté par la formule générale:

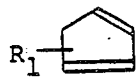

ou

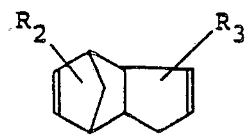

dans laquelle $R_1$ est un atome d'hydrogène ou un groupe alkyle ayant 1 à 3 atomes de carbone et $R_2$ et $R_3$, qui peuvent être semblables ou différents, sont des atomes d'hydrogène ou des groupes alkyle ayant 1 à 3 atomes de carbone, en présence d'un composé catalytique acide.

FIG. 1

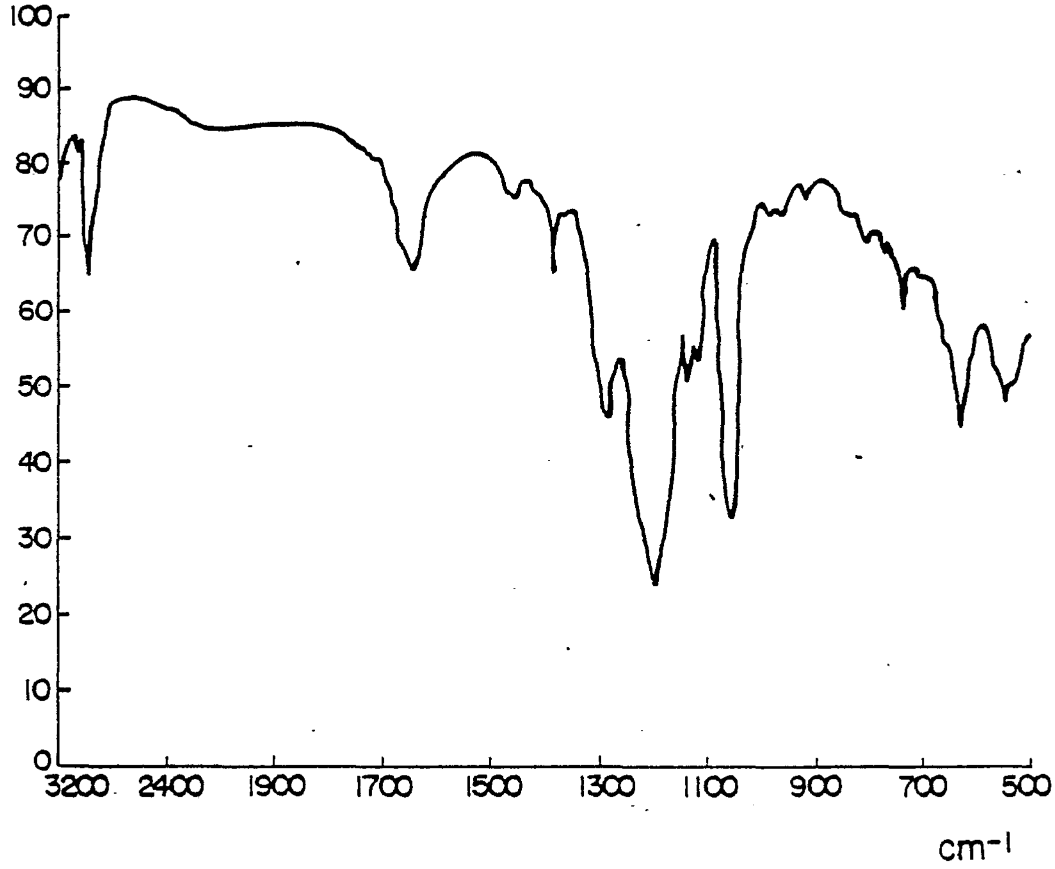
100
90
80
70
60
50
40
30
20
10
0
3200
2400
1900
1700
1500
1300
1100
900
700
500
cm-1

FIG. 2

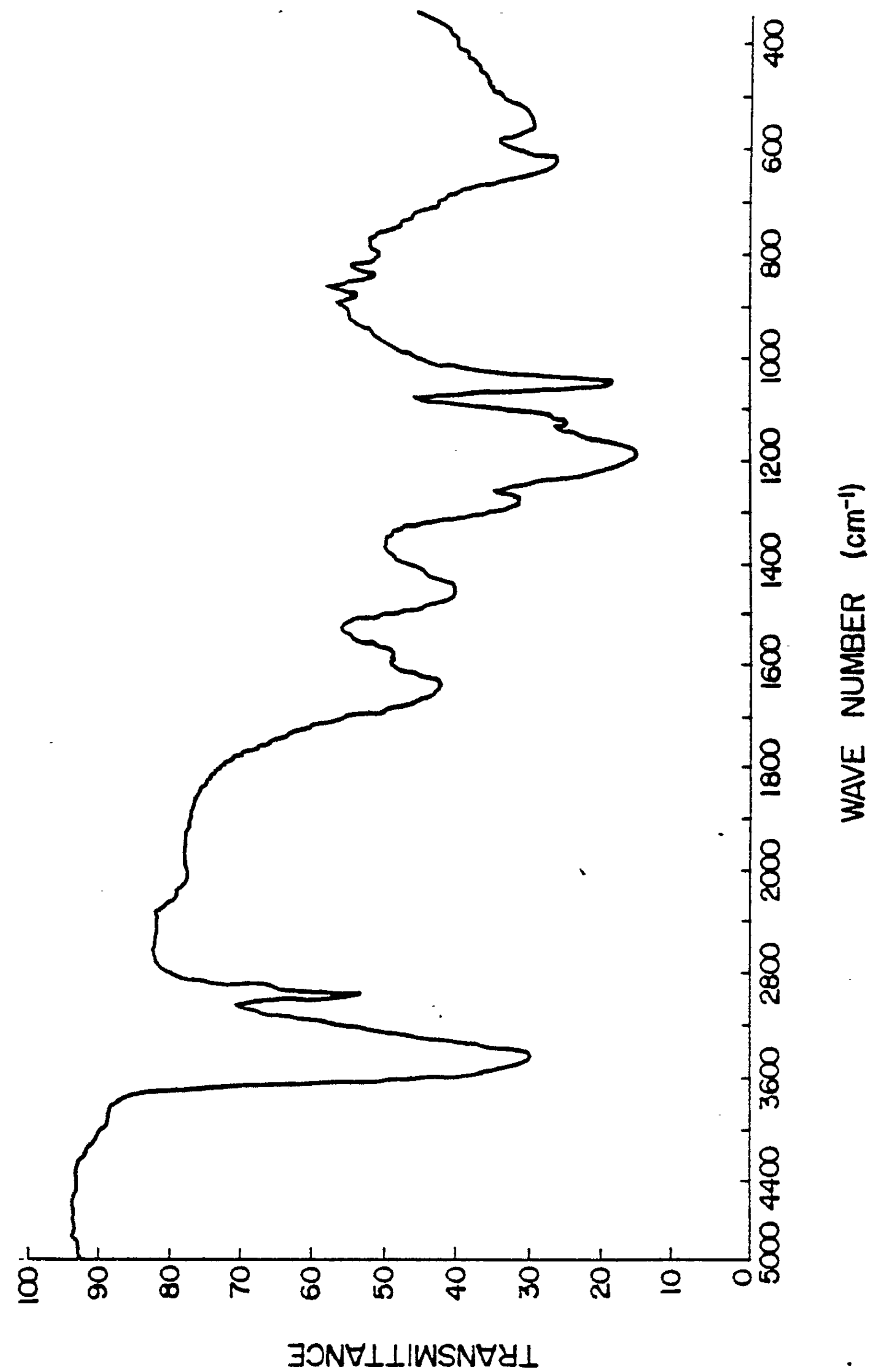
TRANSMITTANCE
100
90
80
70
60
50
40
30
20
10
0
5000
4400
3600
2800
2000
1800
1600
1400
1200
1000
800
600
400
WAVE NUMBER (cm⁻¹)